# EUROPEAN PATENT SPECIFICATION

(11) **EP 3 506 955 B1**
(45) Date of publication and mention of the grant of the patent: **15.07.2020**
(21) Application number: 17767990.9
(22) Date of filing: 01.09.2017
(51) Int. Cl.: A61L 28/00, A61L 31/02, A61L 31/04, A61L 31/06

(54) **A LAYERED FILM**
GESCHICHTETE FOLIE
FILM STRATIFIÉ

(30) Priority: 02.09.2016 DK PA201670674
(43) Date of publication of application: 10.07.2019
(73) Proprietor: Coloplast A/S, 3050 Humblebæk (DK)
(72) Inventor: NIELSEN, Henrik Lindenskov, 2765 Smoerum (DK)
(86) International application number: PCT/DK2017/050279
(87) International publication number: WO 2018/041320

(56) References cited:
- EP-A2- 0 625 343
- WO-A1-2008/014789
- WO-A1-2013/043226
- WO-A1-2017/087809
- WO-A2-2012/016570
- GB-A- 2 201 372
- DATABASE WPI Week 201420 Thomson Scientific, London, GB; AN 2014-E12950 XP002781181, & JP 2014 041900 A (MITSUBISHI PLASTICS IND LTD) 6 March 2014 (2014-03-06)
- DATABASE WPI Week 201634 Thomson Scientific, London, GB; AN 2016-27514B XP002781198, & WO 2016/067727 A1 (TOYOBO KK) 6 May 2016 (2016-05-06)

## Description

The disclosure relates to a layered film having high barrier property and softness.

### Background

Currently available films with desirable high barrier properties have been found to have inferior flexibility or softness properties for certain uses, such as for medical devices. JP2014041900/XP002781181 discloses protective weather resistant film material for solar cells, comprising a moisture-proof film containing a polyester base-material with an inorganic layer on it.

WO2012016570 discloses packages for hydrophilic-coated intermittent urinary catheters, comprising multilayered films with thermoplastic polymer layers and thermoplastic elastomeric polymer layers, with a WVTR of below 0.50 g/m2/mm/24h and an E-modulus of up to 150 MPa, respectively up to 400 MPa. EP0625343 discloses odor barrier material comprising laminates of (co)polymeric material and devices comprising them for use in ostomy and wound healing.

### Brief Summary

The invention is as defined in the claims.

Disclosed is a layered film for use in a medical device comprising a first product component and a second detachable carrier component. The first product component includes a first prime stratum comprising material selected from the group consisting of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof, and a second barrier stratum comprising at least partially inorganic material. An E-modulus (ASTM D882) of the first product component is below 500 MPa and a water vapour transmission rate WVTR (ASTM F1249) of the first product component is below 3 g/m²/24h. The second detachable carrier component comprises material selected from the group consisting of polyethylenes, polypropylenes, polyesters, polyamides, and/or blends thereof. The layered film is configured to have high barrier and softness characteristics and being adapted to endure physical influences, such as stress and strain, during production, handling, storage, transportation and/or application in a final product, such as in a medical device.

### Brief Description of the Drawing

The accompanying drawings are included to provide a further understanding of embodiments and are incorporated into and a part of this specification. The drawings illustrate embodiments and together with the description serve to explain principles of embodiments. Other embodiments and many of the intended advantages of embodiments will be readily appreciated as they become better understood by reference to the following detailed description. The elements of the drawings are not necessarily to scale relative to each other. Like reference numerals designate corresponding similar parts.
Figures 1-5 are schematic cross-sectional views of embodiments of a layered film,
Figure 6 is a schematic view of a sleeve for an intermittent urinary catheter incorporating an embodiment of a layered film,
Figure 7 is a partly sectional top view of an enclosure package for an intermittent urinary catheter incorporating an embodiment of a layered film,
Figure 8 is a schematic top view of a base plate for an ostomy appliance incorporating an embodiment of a layered film,
Figure 9 is a schematic cross-sectional view of a base plate for an ostomy appliance incorporating an embodiment of a layered film,
Figure 10 is a schematic plan view of a bag for collecting bodily waste fluids incorporating an embodiment of a layered film, and
Figure 11 is a schematic cross-sectional view of the ostomy appliance of figure 10 incorporating an embodiment of a layered film.

### Detailed Description

Embodiments provide a layered film comprising a first product component and a second detachable carrier component. Embodiments of the layered film of the disclosure have high barrier and softness characteristics while simultaneously being sufficiently strong to sustain the physical effects of being handled and used in a production set-up. Embodiments of the first product component of the disclosure have high barrier and softness characteristics while simultaneously being sufficiently strong to sustain the physical effects of being handled and used in a production set-up. Embodiments of the layered film of the disclosure are suitable for being incorporated in a medical device. Embodiments provide a layered film having high barrier properties to gases, such as oxygen, carbon dioxide, water vapour and other gases, particularly gases found in flatulence. Embodiments provide a layered film having high barrier properties to liquids and semi-solids, such as water, saline and human body secretions including stool of differing water content. Embodiments provide a layered film having such high barrier properties or characteristics while simultaneously being very soft or flexible.

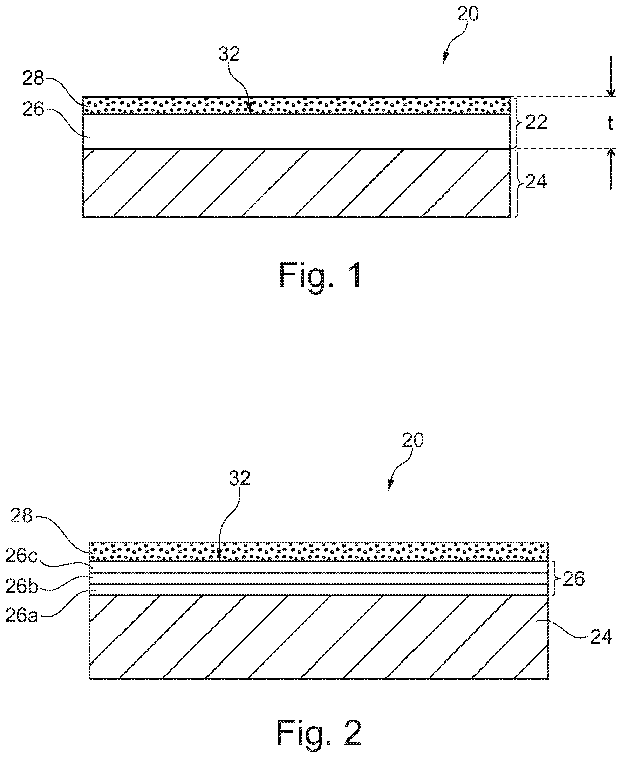

Figure 1 is a schematic cross-sectional view of one embodiment of a layered film 20. The layered film 20 includes a first product component 22 and a second detachable carrier component 24. The first product component 22 includes a first prime stratum 26 and a second barrier stratum 28.

The first prime stratum 26 comprises material selected from the group consisting of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof.

The second barrier stratum 28 comprises at least partially inorganic material. In embodiments, the at least partially inorganic material of the second barrier stratum 28 is selected from a group comprised of metals, nano-coatings, glass-coatings, cured polysilazanes and graphene. These inorganic materials have desirable high barrier properties.

The first product component 22 is adapted to have an E-modulus (Young's modulus), as measured according to American Standard ASTM D882, of below 500 MPa (N/mm²). The first product component 22 is adapted to have a water vapour transmission rate (WVTR), as measured according to ASTM F1249, of below 3 g/m²/24h (three grams per square meter per 24 hour period).

Figure 2 is a schematic cross-sectional view of one embodiment of a layered film 20 in which the first prime stratum 26 comprises a plurality of individual layers 26a, 26b, 26c. Each of the individual layers 26a, 26b, 26c comprises one or more materials selected from a group comprised of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof.

The second detachable carrier component 24 comprises material selected from the group consisting of polyethylenes, polypropylenes, polyesters, polyamides, and/or blends thereof.

In embodiments, a second E-modulus of the second detachable carrier component 24 is greater than a first E-modulus of the first product component 22.

In embodiments, the layered film 20 includes one or more layers of a support material having a suitable mechanical stability, providing additional support to the layered film 20. In embodiments, the support material has mechanical stability at temperatures above 70°C. In embodiments, the one or more layers of support material is/are included in the first product component 22. In embodiments, the one or more layers of support material is/are included in the second detachable carrier component 24. In embodiments, one or more layers of support material is/are included in each of the first product component 22 and the second detachable carrier component 24. In embodiments, the one or more layers of support material include high-density polyethylenes (HDPE), polypropylenes (PP) and polyesters (PET).

The one or more layers of support material assist(s) in providing for the layered film 20 to better endure stress and strain during production, handling, storage, transportation and/or application in a product or device incorporating the first product component 22. Particularly, but not exclusively, the support material provides for increased support during a production process, in which the second barrier stratum 28 is provided as a coating.

Embodiments according to the above, provide a layered film 20 having a high level of barrier properties stemming from the material of the second barrier stratum 28, while simultaneously being more suitable for general handling and for inclusion in further production steps. These improvements particularly provide for the incorporation of a thin, at least partially inorganic barrier material having high barrier properties, to be combined with a material layer of high flexibility and softness properties and being handled and worked.

This is advantageous in production of medical devices. Advantages of the embodiments of the present disclosure include the provision of a film material suitable particularly for medical devices that has high barrier properties and being processible throughout a full (complete) processing cycle of a medical device.

With currently available films, the inclusion of high barrier property materials requires a relatively stiff or rigid substrate, such as oriented polypropylene or polyesters, because these high barrier property materials typically are very sensible to elongation and/or bending. Unlike these currently available film materials having high barrier properties, the first product component 22 of the layered film 20 according to the disclosure can be made very flexible and/or soft, because the second detachable carrier component 24 provides the layered film 20 with a favourable robustness for further process steps, such as including the manufacture of a medical device. Moreover, in embodiments, once one or more steps in a production process of a product demanding a robustness of the layered film 20 disclosed in this application is finalized, the second detachable carrier component 24 can be removed. Accordingly, the layered film 20 of the disclosure offers a solution for combining materials having very high barrier properties with materials having a high degree of flexibility and/or softness. This is found to be very advantageous for use in the production of various products, including medical devices.

Subject to dependency on the production process and/or the type of product incorporating the first product component 22 of the layered film 20, the second detachable carrier component 24 can be detached, or removed, during manufacture of the product, before or after storage of the layered film 20, before or after handling the layered film 20 in a production process of a product incorporating the first product component 22, or by a user of a product, such as a medical device in a situation when he or she prepares the medical device for use.

Figures 1 and 2 further illustrate embodiments wherein the second barrier stratum 28 is attached to a primary surface 32 of the first prime stratum 26.

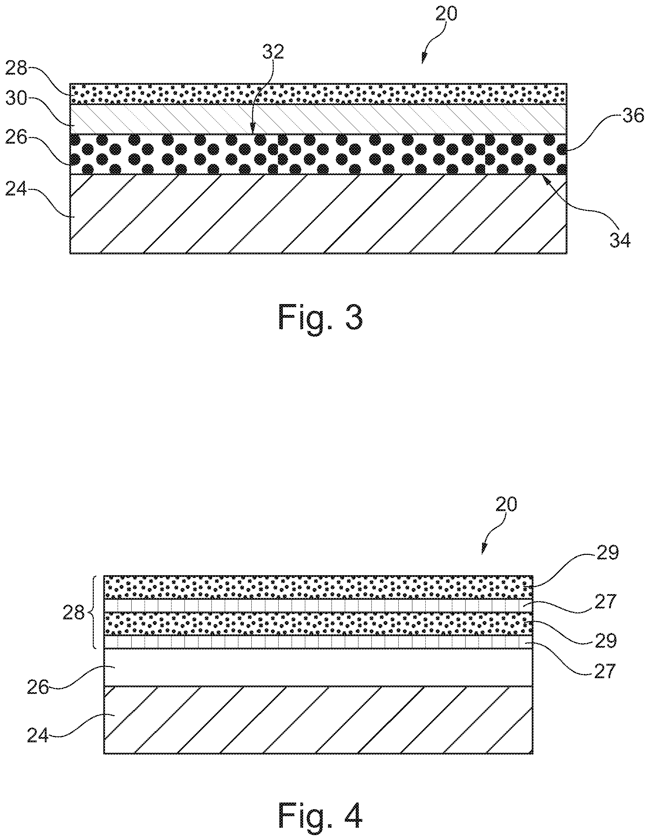

Figure 3 is a schematic cross-sectional view illustrating one embodiment of the layered film 20 wherein the layered film 20 includes a third binder stratum 30 provided between the first prime stratum 26 and the second barrier stratum 28. In embodiments, the third binder stratum 30 helps to provide a reinforced attachment of the second barrier stratum 28 to the first prime stratum 26. In embodiments, the third binder stratum 30 comprises material selected from a group consisting of polyethylenes, polyethylene copolymers, polypropylene copolymers, thermoplastic polyurethanes, thermoplastic polyester elastomers, thermoplastic polyamide elastomers, thermoplastic elastomer compounds and/or blends thereof.

In other embodiments, the third binder stratum 30 comprises material selected from a group comprising polymers having one or more functional side-groups. In embodiments, the functional side-groups comprise nitiriles, carboxylates, acid anhydrides, aldehydes, ketones, alcohols, amines, esters, amides, carboxylic acids, haloalkanes and ethers. These materials are found to work well in providing attachment between the first prime stratum 26 and the second barrier stratum 28.

Figure 3 further illustrates an embodiment of the layered film 20 wherein the third binder stratum 30 is attached to the primary surface 32 of the first prime stratum 26 and the second barrier stratum 28 is attached to the third binder stratum 30.

In other embodiments, the primary surface 32 of the first prime stratum 26 is pre-treated by corona treatment, flame treatment or plasma treatment instead of providing the third binder stratum 30. The pre-treatment can advantageously be carried out in a separate process before application of the second barrier stratum 28. In this alternative manner, the second barrier stratum 28 can be attached directly to the primary surface 32. In embodiments, this provides a simple production process and simple intermediate product.

In an embodiment, the layered film 20 includes one or more additives selected from a group comprising antioxidants, colorants, processing additives, release additives and anti-block additives. In figure 3, an additive is indicated at 36 as being incorporated in the first prime stratum 26. However, it is to be understood that the one or more additives may be incorporated in any of the strata of the first product component 22. In other embodiments, one or more additives 36 is provided in one or more additional additive stratum/strata (not shown).

In embodiments, the second detachable carrier component 24 is detachably attached to a secondary surface 34 of the first prime stratum 26. In one definition, "detachably attached" is intended to reflect that the second detachable carrier component 24 can be removed from the secondary surface 34 of the first prime stratum 26 without breaking or damaging the first product component 22 in any way. Moreover, in an additional or alternative definition, the detachable attachment means that only a relatively small force is necessary to separate the components.

In embodiments, the second detachable carrier component 24 and the first prime stratum 26 are configured to have some compatibility, or affinity, with each other, meaning that weak chemical forces between them tend to bind them to each other. In embodiments, a degree of attachment between the second detachable carrier component 24 and the first prime stratum 26 is controlled in the production process, such as by choice of polymers of the materials, processing parameters and functional additives. In embodiments, the materials for the second detachable carrier component 24 and the first prime stratum 26, and/or the process parameters, are configured such that the second detachable carrier component 24 and the first prime stratum 26 are incapable of attaching permanently to each other.

In embodiments, the second detachable carrier component 24 is attached with a weak adhesive. In embodiments, the second detachable carrier component 24 is attached only to a portion, or portions, of the secondary surface 34, such as in a plurality of distinct zones each providing a relatively small contact area between the surfaces. Other ways of detachably or releasably attaching the second detachable carrier component 24 to the secondary surface 34 of the first prime stratum 26 are acceptable, provided that they correspond to the definitions set out above.

In embodiments, the second barrier stratum 28 is provided as a coating. Particularly, but not exclusively, this provides for the second barrier stratum 28 to be very thin while also being of a material having high, or even extremely high, barrier properties such as the at least partially inorganic materials mentioned elsewhere in this disclosure. In embodiments, the coating of the second barrier stratum 28 can be applied to the primary surface 32 of the first prime stratum 26 or to the third binder stratum 30 by way of physical or chemical vapor deposition, spraying, roll-to-roll coating or other chemical, electrochemical or mechanical techniques, including co-extrusion of the barrier and substrate materials.

In embodiments, a thickness t (figure 1) of the first product component 22 is below 100 µm. The thickness t of the first product component 22 will naturally influence the E-modulus of the first product component 22. A plurality of different embodiments having a combined thickness t of the first prime stratum 26 and the second barrier stratum 28 of below 100 µm, are provided by varying the distribution of individual thicknesses of the first prime stratum 26 and the second barrier stratum 28. In embodiments, a thickness of the first prime stratum 26 is within a range of 10-90 µm, such as 20-80 µm, such as 30-70 µm, such as 40-60 µm, such as 50 µm. Depending on the desired particular characteristics of the first product component 22, the individual thicknesses of the first prime stratum 26 and the second barrier stratum 28 are chosen such as to also fulfil the limitation of the combined thickness of less than 100 µm. In other words, in embodiments, the thickness of the second barrier stratum 28 can be varied thereby also providing a "sub-range" of barrier property options for very soft, high barrier property films suitable for further production, such as for medical devices.

In embodiments, an oxygen transmission rate OTR, as measured according to American Standard ASTM D3985, of the first product component 22, is below 100 cm³/m²/24h. Particularly, but not exclusively, a thickness of the second barrier stratum 28 can be adapted to make the first product component 22 meet the OTR limitation of 100 cm³/m²/24h. Providing the first product component 22 with this characteristic presents a film particularly well suited for the purpose of being used for the walls of a collecting bag for bodily waste fluids, such as a bag for collecting fecal matter or urine.

In embodiments, the first prime stratum 26 of the first product component 22 is manufactured by a cast film extrusion process. In other embodiments, the first prime stratum 26 of the first product component 22 is manufactured by a blown film extrusion process. In cases where in the first product component 22 of the layered film 20 is applied in a product, such as a medical device, the film extrusion process can be chosen according to product requirements. Blown films typically are tougher and more resistant to puncture than cast films. On the other hand, cast films are typically clear and transparent, allowing them to be used for enclosing products where visual identification is of importance. In embodiments, the first prime stratum 26 includes a blown film particularly suitable for a collecting bag for bodily waste fluids. In another embodiment, the first prime stratum 26 includes a cast film particularly suitable for sleeve for an intermittent, urinary catheter. In yet other embodiments, the first prime stratum 26 includes a blown film particularly suitable for sleeve for an intermittent, urinary catheter.

In embodiments, the third binder stratum 30 comprises material selected from a group consisting of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof. These materials are found to work well in providing attachment between the first prime stratum 26 and the second barrier stratum 28.

Figure 4 is a schematic cross-sectional view of one embodiment of a layered film 20, in which the second barrier stratum 28 includes one or more protective layers 29 selected from a group comprising polymers coating, nano-coatings and polysilazane coatings. The protective layer(s) 29 provide(s) additional security against damaging of the barrier material 27 of the second barrier stratum 28 during production and use.

In embodiments, the coating of the second barrier stratum 28 includes a plurality of individual barrier layers. Figure 4 further illustrates embodiments, wherein the plurality of individual barrier layers 27 are interchangeably, or alternatingly, arranged with a plurality of protective layers 29. In one embodiment, the second barrier stratum 28 is provided as a laminate consisting of multiple, at least partially inorganic barrier materials 27, selected from a group comprised of metals, ceramics, nano-coatings, cured polysilazanes, and graphene, and one or more protective layers 29 selected from a group comprising polymers coating, nano-coatings and cured polysilazane coatings. In embodiments, the detrimental effects of pinholes inevitably forming in the coating surface, are minimized while the flexibility of the first prime stratum 26 is not compromised. Thereby, such laminate configuration of the second barrier stratum 28 is found to provide a particularly robust second barrier stratum 28, particularly advantageous in the production of films having high barrier properties and being very thin and soft.

In embodiments, suitable metals for the second barrier stratum 28 include aluminium, chromium, copper and others. Suitable ceramic materials include oxides, such as, but not limited to, silicon oxides (SiOx), aluminium oxides (AlOx) and titanium oxides (TiOx). Suitable nano-coatings include Ormocer®s, a material class of inorganic-organic hybrid polymers based on chemical nanotechnology, available from Fraunhofer, Germany. Other suitable nano-coatings include Tera-Barrier from Tera Barrier Films, ltd., Singapore and SunBar® from SunChemicals®, The Netherlands. Suitable polysilazanes include both perhydropolysilazanes (inorganic) and organopolysilazanes (organic) cured by treatment with water (H2O) and heat, or cured by ultraviolet (UV) radiation. In embodiments, the coating of the second barrier stratum 28 includes a Ceramis® coating, available from Amcor, ltd., Australia.

In embodiments, the second barrier stratum 28 includes a nano-clay. In embodiments, the second barrier stratum 28 includes one or more plate shaped filler materials.

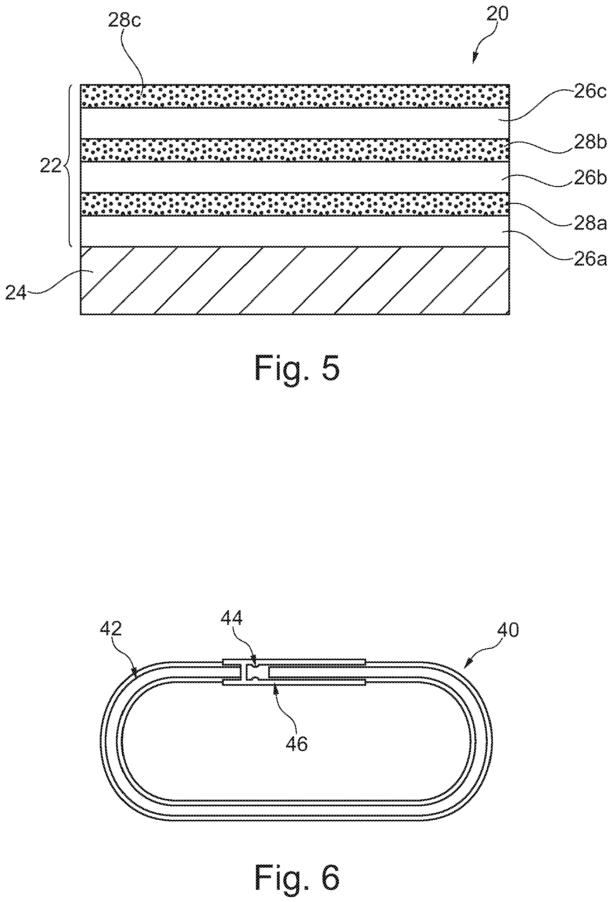

Figure 5 is a schematic cross-sectional view of one embodiment of a layered film 20, in which the first product component 22 of the layered film 20 includes a plurality of first prime strata 26a, 26b, 26c, a plurality of second barrier strata 28a, 28b, 28c and optionally a plurality of third binder strata 30 (not shown) alternatingly arranged. These embodiments provide further options for high barrier property films as well as facilitating more robust products.

Figure 6 is a schematic view of a sleeve 40 for an intermittent urinary catheter 42, the sleeve 40 comprising the first product component 22 of the layered film 20 disclosed herein. In embodiments, the sleeve 40 defines at least a portion of an openable compartment 44 for storing the catheter 42. In embodiments, the catheter 42 is coated with a hydrophilic coating. In embodiments, the sleeve 40 is configured to also contain a component for activation of the hydrophilic coating on the catheter 42. In embodiments, the component for activation of the hydrophilic coating on the catheter 42 is a liquid 46.

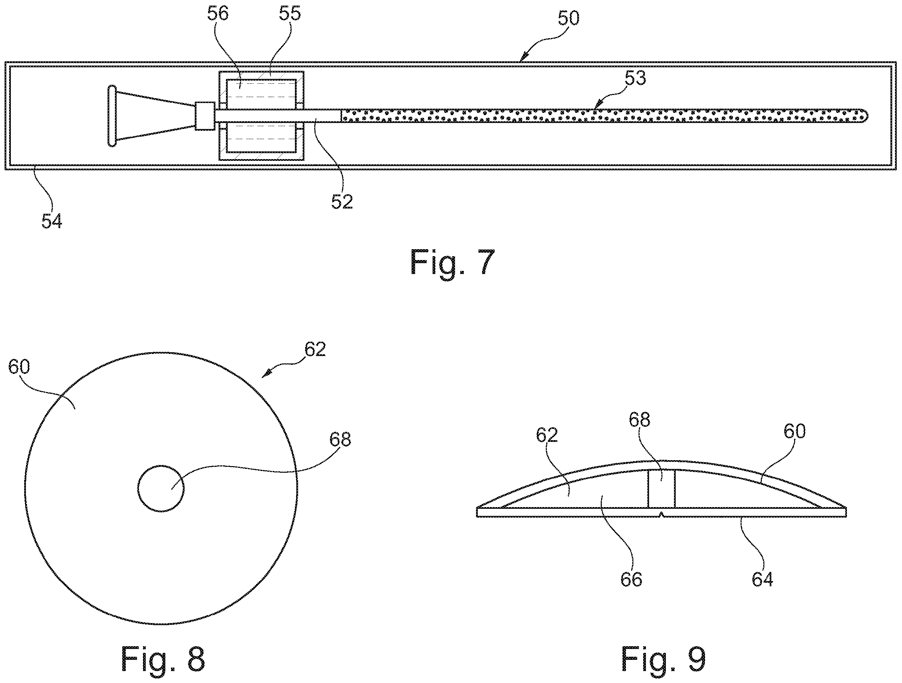

Figure 7 is a partly sectional top view of an enclosure package 50 for an intermittent urinary catheter 52, the enclosure package 50 comprising the first product component 22 of the layered film 20 disclosed herein. In embodiments, the enclosure package 50 defines at least a portion of an openable compartment 54 for storing the catheter 52. In embodiments, the catheter 52 is coated with a hydrophilic coating 53. In embodiments, the enclosure package 50 is configured to also contain a component 55 for activation of the hydrophilic coating 53 on the catheter 52. In embodiments, the component 55 for activation of the hydrophilic coating 53 on the catheter 52 comprised a liquid 56.

Figure 8 is a schematic top view, and figure 9 is a schematic cross-sectional view of a base plate 62 for an ostomy appliance, the base plate 62 including one or more layers 60 of the first product component 22 of the of the layered film 20 disclosed herein. In embodiments, the base plate 62 includes an adhesive material 66 supported by the layer 60 and protected prior to application by a release liner 64. An ostomy receiving opening 68 is also illustrated.

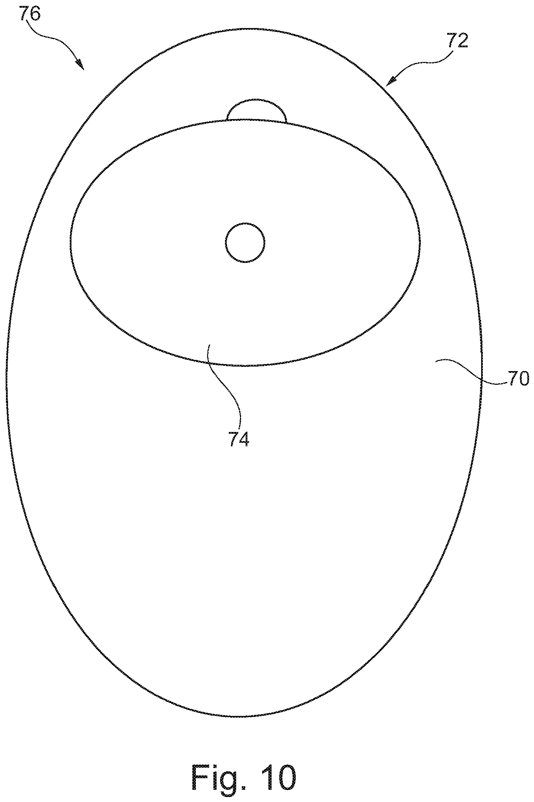

Figure 10 is a schematic plan view of a bag 72 for collecting bodily waste fluids, the collecting bag 72 comprising one or more layers 70 of the first product component 22 of the layered film 20 disclosed herein. In embodiments, the collecting bag 72 is attached to a base plate 74 to form an ostomy appliance 76.

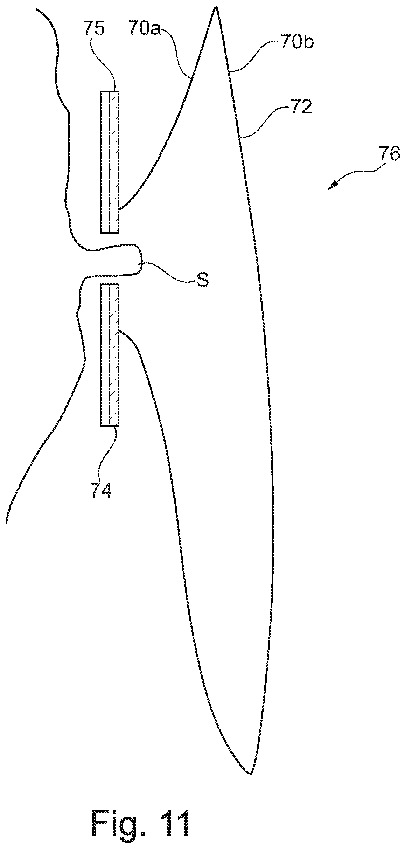

Figure 11 is a schematic cross-sectional view of the ostomy appliance 76 of figure 10 including two layers 70a, 70b of the first product component 22 forming a bag 72 for collecting stomal wastes exiting a stoma S. In embodiments, the layer 70a of the collecting bag 72 is attached to a top film layer 75 of the base plate 74. In embodiments, the top film layer 75 includes the first product component 22 of the layered film 20.

Further disclosed is a backing component for a wound dressing, the backing component comprising one or more layers of the first product component 22 of the layered film 20.

## Claims

1. A layered film for use in a medical device comprising a first product component and a second detachable carrier component, the first product component comprising:
- a first prime stratum comprising material selected from the group consisting of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof; and
- a second barrier stratum comprising at least partially inorganic material,
wherein an E-modulus (ASTM D882) of the first product component is below 500 MPa and a water vapour transmission rate WVTR (ASTM F1249) of the first product component is below 3 g/m²/24h, and
wherein
- the second detachable carrier component comprises material selected from the group consisting of polyethylenes, polypropylenes, polyesters, polyamides, and/or blends thereof.

2. The film of claim 1, further comprising a third binder stratum provided between the first prime stratum and the second barrier stratum.

3. The film of claim 1, wherein the second barrier stratum is attached to a primary surface of the first prime stratum.

4. The film of claim 2, wherein the third binder stratum is attached to a primary surface of the first prime stratum and the second barrier stratum is attached to the third binder stratum.

5. The film of claim 1, wherein the second detachable carrier component is detachably attached to a secondary surface of the first prime stratum.

6. The film of claim 1, wherein the at least partially inorganic material of the second barrier stratum is selected from a group comprising metals, ceramics, nano-coatings, glass-coatings, cured polysilazanes and graphene.

7. The film of claim 1, wherein the second barrier stratum is provided as a coating.

8. The film of claim 1, wherein a thickness of the first product component is below 100 µm.

9. The film of claim 1, wherein an oxygen transmission rate OTR (ASTM D3985) of the first product component is below 100 cm³/m²/24h.

10. The film of claim 2, wherein the third binder stratum comprises material selected from the group consisting of polyethylenes, polyethylene copolymers, polypropylene copolymers, thermoplastic polyurethanes, thermoplastic polyester elastomers, thermoplastic polyamide elastomers, thermoplastic elastomer compounds, and/or blends thereof.

11. The film of claim 2, wherein the third binder stratum comprises a material selected from a group comprising polymers having one or more functional side-groups.

12. The film of claim 1, wherein the second barrier stratum further comprises one or more protective layers selected from a group comprising polymer coatings, nano-coatings and cured polysilazane coatings.

13. The film of claim 1, wherein the second barrier stratum is provided as a laminate consisting of multiple layers the materials of which alternate between at least partially inorganic barrier materials, selected from a group comprised of metals, ceramics, nano-coatings, cured polysilazanes and graphene, and one or more protective layers selected from a group comprising polymers coating, nano-coatings and cured polysilazane coatings.

14. The film of claim 1, further comprising one or more additives selected from a group comprising antioxidants, colorants, processing additives, release additives and anti-block additives.

15. The film of claim 15, wherein the one or more additives is/are provided in one or more additional stratum/strata.

16. The film of any one of claims 1 or 2, wherein the first product component comprises a plurality of first prime strata, second barrier strata and optionally third binder strata alternatingly arranged.

17. The film of claim 1, wherein a first E-modulus of the detachable carrier component is greater than a second E-modulus of the first product component.

18. The film of claim 1, further comprising a support material having mechanical stability at temperatures above 70°C.

## Patentansprüche

1. Geschichteter Film zur Verwendung in einer medizinischen Vorrichtung, umfassend eine erste Produktkomponente und eine zweite, ablösbare Trägerkomponente, wobei die erste Produktkomponente umfasst:
- eine erste Hauptschicht, umfassend Material ausgewählt aus der Gruppe bestehend aus Polyethylenen, Polyethylen-Copolymeren, Polypropylen-Copolymeren, thermoplastischen Polyurethanen, thermoplastischen Polyester-Elastomeren, thermoplastischen Polyamid-Elastomeren, thermoplastischen Elastomerverbindungen und/oder Gemischen davon; und
- eine zweite Barriereschicht, umfassend wenigstens teilweise anorganisches Material,
wobei der E-Modul (ASTM D882) der ersten Produktkomponente weniger als 500 MPa beträgt und die Wasserdampfdurchlässigkeitsrate WVTR (ASTM F1249) der ersten Produktkomponente weniger als 3 g/m²/24 h beträgt, und
wobei
- die zweite, ablösbare Trägerkomponente Material ausgewählt aus der Gruppe bestehend aus Polyethylenen, Polypropylenen, Polyestern, Polyamiden und/oder Gemischen davon umfasst.

2. Film gemäß Anspruch 1, ferner umfassend eine dritte Bindeschicht, die zwischen der ersten Hauptschicht und der zweiten Barriereschicht angeordnet ist.

3. Film gemäß Anspruch 1, wobei die zweite Barriereschicht an einer Hauptoberfläche der ersten Hauptschicht angeordnet ist.

4. Film gemäß Anspruch 2, wobei die dritte Bindeschicht an einer Hauptoberfläche der ersten Hauptschicht angebracht ist und die zweite Barriereschicht an der dritten Bindeschicht angebracht ist.

5. Film gemäß Anspruch 1, wobei die zweite, ablösbare Trägerkomponente ablösbar an einer zweiten Oberfläche der ersten Hauptschicht angebracht ist.

6. Film gemäß Anspruch 1, wobei das wenigstens teilweise anorganische Material der zweiten Barriereschicht ausgewählt ist aus der Gruppe umfassend Metalle, Keramik, Nanobeschichtungen, Glasbeschichtungen, gehärtete Polysilazane und Graphen.

7. Film gemäß Anspruch 1, wobei die zweite Barriereschicht als Beschichtung bereitgestellt ist.

8. Film gemäß Anspruch 1, wobei eine Dicke der ersten Produktkomponente weniger als 100 µm beträgt

9. Film gemäß Anspruch 1, wobei die Sauerstoffdurchlässigkeitsrate OTR (ASTM D3985) der ersten Produktkomponente weniger als 100 cm³/m²/24 h beträgt.

10. Film gemäß Anspruch 2, wobei die dritte Bindeschicht Material ausgewählt aus der Gruppe bestehend aus Polyethylenen, Polyethylen-Copolymeren, Polypropylen-Copolymeren, thermoplastischen Polyurethanen, thermoplastischen Polyester-Elastomeren, thermoplastischen Polyamid-Elastomeren, thermoplastischen Elastomerverbindungen und/oder Gemischen davon umfasst.

11. Film gemäß Anspruch 2, wobei die dritte Bindeschicht ein Material ausgewählt aus einer Gruppe umfassend Polymere mit einer oder mehreren funktionellen Seitengruppen umfasst.

12. Film gemäß Anspruch 1, wobei die zweite Barriereschicht ferner eine oder mehrere Schutzschichten ausgewählt aus einer Gruppe umfassend Polymerbeschichtungen, Nanobeschichtungen und gehärtete Polysilazanbeschichtungen umfasst.

13. Film gemäß Anspruch 1, wobei die zweite Barriereschicht als Laminat bereitgestellt ist, bestehend aus mehreren Schichten der Materialien, die zwischen wenigstens teilweise anorganischen Barrierematerialien ausgewählt aus einer Gruppe bestehend aus Metallen, Keramik, Nanobeschichtungen, gehärteten Polysilazanen und Graphen, und einer oder mehreren Schutzschichten ausgewählt aus einer Gruppe umfassend Polymerbeschichtungen, Nanobeschichtungen und gehärtete Polysilazanbeschichtungen abwechseln.

14. Film gemäß Anspruch 1, ferner umfassend einen oder mehrere Zusatzstoffe ausgewählt aus einer Gruppe umfassend Antioxidationsmittel, Farbstoffe, Verarbeitungszusatzstoffe, Trennmittelzusatzstoffe und Antiblockierzusatzstoffe.

15. Film gemäß Anspruch 15, wobei der eine oder die mehreren Zusatzstoffe in einer oder mehreren zusätzlichen Schicht/Schichten bereitgestellt ist/sind.

16. Film gemäß einem der Ansprüche 1 oder 2, wobei die erste Produktkomponente eine Vielzahl von ersten Hauptschichten, zweiten Barriereschichten und gegebenenfalls dritten Bindeschichten, die abwechselnd angeordnet sind, umfasst.

17. Film gemäß Anspruch 1, wobei ein erster E-Modul der ablösbaren Trägerkomponente größer als ein zweiter E-Modul der ersten Produktkomponente ist.

18. Film gemäß Anspruch 1, ferner umfassend ein Trägermaterial mit mechanischer Stabilität bei Temperaturen von über 70 °C.

## Revendications

1. Film stratifié destiné à une utilisation dans un dispositif médical comprenant un premier élément constitutif produit et un deuxième élément constitutif support amovible, le premier élément constitutif produit comprenant :
- une première couche primaire comprenant un matériau sélectionné dans le groupe constitué de polyéthylènes, de copolymères de polyéthylène, de copolymères de polypropylène, de polyuréthanes thermoplastiques, d'élastomères de polyesters thermoplastiques, d'élastomères de polyamides thermoplastiques, de composés élastomères thermoplastiques, et/ou de mélanges de ceux-ci ; et
- une deuxième couche barrière comprenant un matériau au moins en partie inorganique,
dans lequel un module d'élasticité (ASTM D882) du premier élément constitutif produit est inférieur à 500 MPa et un coefficient de transmission de la vapeur d'eau CTVE (ASTM F1249) du premier élément constitutif produit est inférieur à 3 g/m²/24 h, et
dans lequel
le deuxième élément constitutif support amovible comprend un matériau sélectionné dans le groupe constitué de polyéthylènes, de polypropylènes, de polyesters, de polyamides et/ou de mélanges de ceux-ci.

2. Film selon la revendication 1, comprenant en outre une troisième couche de liant disposée entre la première couche principale et la deuxième couche barrière.

3. Film selon la revendication 1, dans lequel la deuxième couche barrière est fixée à une surface principale de la première couche primaire.

4. Film selon la revendication 2, dans lequel la troisième couche de liant est fixée à une surface principale de la première couche primaire et la deuxième couche barrière est fixée à la troisième couche de liant.

5. Film selon la revendication 1, dans lequel le deuxième élément constitutif support amovible est fixé de façon amovible à une surface secondaire de la première couche primaire.

6. Film selon la revendication 1, dans lequel le matériau au moins en partie inorganique de la deuxième couche barrière est sélectionné dans un groupe comprenant des métaux, des céramiques, des nano-revêtements, des revêtements de verre, des polysilazanes durcis et le graphène.

7. Film selon la revendication 1, dans lequel la deuxième couche barrière est présente sous la forme d'un revêtement.

8. Film selon la revendication 1, dans lequel une épaisseur du premier élément constitutif produit est inférieure à 100 µm.

9. Film selon la revendication 1, dans lequel un coefficient de transmission de l'oxygène CTO (ASTM D3985) du premier élément constitutif produit est inférieur à 100 cm³/m²/24 h.

10. Film selon la revendication 2, dans lequel la troisième couche de liant comprend un matériau sélectionné dans le groupe constitué de polyéthylènes, de copolymères de polyéthylène, de copolymères de polypropylène, de polyuréthanes thermoplastiques, d'élastomères de polyesters thermoplastiques, d'élastomères de polyamides thermoplastiques, de composés élastomères thermoplastiques, et/ou de mélanges de ceux-ci.

11. Film selon la revendication 2, dans lequel la troisième couche de liant comprend un matériau sélectionné dans un groupe comprenant des polymères comportant un ou plusieurs groupes latéraux fonctionnels.

12. Film selon la revendication 1, dans lequel la deuxième couche barrière comprend en outre une ou plusieurs couches de protection sélectionnées dans un groupe comprenant des revêtements de polymères, des nano-revêtements et des revêtements de polysilazane durci.

13. Film selon la revendication 1, dans lequel la deuxième couche barrière est présente sous la forme d'un stratifié constitué de plusieurs couches dont les matériaux alternent entre des matériaux de barrière au moins en partie inorganiques, sélectionnés dans un groupe constitué de métaux, de céramiques, de nano-revêtements, de polysilazanes durcis et du graphène, et une ou plusieurs couches de protection sélectionnées dans un groupe comprenant des revêtements de polymères, des nano-revêtements et des revêtements de polysilazane durci.

14. Film selon la revendication 1, comprenant en outre un ou plusieurs additifs sélectionnés dans un groupe comprenant des antioxydants, des colorants, des additifs de traitement, des additifs anti-adhérents et des additifs anti-adhésion.

15. Film selon la revendication 15, dans lequel lesdits un ou plusieurs additifs sont présents dans une ou plusieurs couches additionnelles.

16. Film selon l'une quelconque des revendications 1 ou 2, dans lequel le premier élément constitutif produit comprend une pluralité de premières couches principales, deuxièmes couches barrières et optionnellement troisièmes couches de liant disposées en alternance.

17. Film selon la revendication 1, dans lequel un premier module d'élasticité de l'élément constitutif support amovible est supérieur à un deuxième module d'élasticité du premier élément constitutif produit.

18. Film selon la revendication 1, comprenant en outre un matériau de support ayant une stabilité mécanique à des températures supérieures à 70 °C.
